# EUROPÄISCHE PATENTSCHRIFT

(11) **EP 0 915 817 B1**
(45) Veröffentlichungstag und Bekanntmachung des Hinweises auf die Patenterteilung: **13.06.2001**
(21) Anmeldenummer: 97935531.0
(22) Anmeldetag: 21.07.1997
(51) Int. Cl.: C07C 29/78, C07C 31/26

(54) **VERFAHREN ZUR HERSTELLUNG VON KRISTALLINEM D-SORBIT**
METHOD OF PRODUCING CRYSTALLINE D-SORBITOL
PROCEDE POUR PRODUIRE DU D-SORBITOL CRISTALLIN

(30) Priorität: 23.07.1996 DE 19629640
(43) Veröffentlichungstag der Anmeldung: 19.05.1999
(73) Patentinhaber: mg technologies ag, 60325 Frankfurt am Main (DE)
(72) Erfinder: GUTMANN, Pedro, D-61476 Kronberg (DE); WIESENBART, Jakob, D-65510 Idstein (DE)
(86) Internationale Anmeldenummer: EP9703924
(87) Internationale Veröffentlichungsnummer: WO9803457

(56) Entgegenhaltungen:
- EP-A- 0 032 288
- EP-A- 0 330 352
- EP-A- 0 528 604
- EP-A- 0 529 852
- DE-A- 2 350 619
- DE-A- 3 732 141

## Beschreibung

Die Erfindung betrifft ein Verfahren zur Herstellung von kristallinem D-Sorbit durch Eindampfung einer wäßrigen D-Sorbit-Lösung im Vakuum und nachfolgende Schmelzkristallisation der bei der Eindampfung erhaltenen D-Sorbit-Schmelze.

D-Sorbit gehört zu den 6-wertigen Alkoholen und hat die Summenformel C₆H₁₄O₆. D-Sorbit wird aus D-Glukose durch elektrolytische Reduktion oder katalytische Hydrierung hergestellt. D-Sorbit hat einen süßen Geschmack und wird als Zuckerersatzstoff sowie in der kosmetischen und pharmazeutischen Industrie sowie zur Herstellung von Polyethern und Tensiden verwendet. D-Sorbit kristallisiert in der α-, β- und γ-Modifikation, wobei die γ-Modifikation die thermodynamisch stabile Form ist. D-Sorbit wird entweder in Form konzentrierter wäßriger Lösungen oder in fester kristalliner Form als industrieller Rohstoff verwendet. Um den festen kristallinen D-Sorbit gut handhaben zu können, wird angestrebt, daß er möglichst weitgehend in der γ-Modifikation vorliegt, da diese Form nicht nur thermodynamisch stabil sondern auch vergleichsweise wenig hygroskopisch ist.

Aus der DE-A 32 45 170 ist ein Verfahren zur Herstellung von Sorbit mit verbesserten Tablettierungseigenschaften bekannt, bei dem eine Lösung von kristallisierter Glukose bei einer Temperatur unterhalb 170°C hydriert und die so erhaltene Sorbit-Lösung bei einer Temperatur von 140 bis 170°C so sprühgetrocknet wird, daß das erhaltene Produkt einen Wassergehalt von weniger als 1 % aufweist.

Aus der DE-C 23 50 619 ist ein Verfahren zur kontinuierlichen Herstellung von kristallisiertem Sorbit durch Einbringen von geschmolzenem und körnigem Sorbit in einen Behälter, in dem die so erhaltene Masse bei einer erhöhten Temperatur in Bewegung gehalten wird, bekannt. Bei diesem Verfahren wird zerstäubter Sorbit oder Sorbit in Form von Tröpfchen, Bahnen, Schleiern oder Streifen mit über 90 % Trockensubstanz im geschmolzenen Zustand fortlaufend mit 20 bis 80 Gew% Sorbit-Pulver mit einer Körnung unter 5 mm in den Behälter eingebracht und die so erhaltene Masse durch Rotieren des offenen Behälters mit waagerechter oder zur Waagerechten geneigten Achse umgewälzt, wobei auf die Oberfläche der umgewälzten Masse geschmolzener Sorbit und Sorbit-Pulver aufgetragen wird. Die umgewälzte Masse wird auf einer Temperatur von über 90°C gehalten und die sich hauptsächlich auf der Oberfläche der umgewälzten Masse absondernden größeren Körner werden durch Überfließen am Ausgang des rotierenden

Behälters aufgefangen sowie anschließend einer Reifungsstufe zum Auskristallisieren des Sorbits unterworfen. Der Schmelzkristallisation ist ein Gefäß für die Vakuumverdampfung vorgeschaltet, das sich dazu eignet, eine Sorbit-Lösung auf einen Trockengehalt von über 98 Gew% zu bringen.

Aus der EP-A 0032288 ist ein modifizierter γ- Sorbit mit vorteilhaften Tablettierungseigenschaften bekannt, der einen Schmelzpunkt von 100 bis 101 °C hat und der einen kristallinen Anteil > 90% aufweist. Die Herstellung dieses Produkts erfolgt durch Schmelzkristallisation in einer Mischvorrichtung, der die Schmelze bei 96 bis 97 °C zugeführt wird, in der die Schmelze geknetet und gekühlt wird und der ein heißer Luftstrom zur Verhinderung einer vorzeitigen Kristallisation zugeführt wird.

Die EP-A 0330352 offenbart ein Verfahren zur Herstellung von festem Sorbit, bei dem Impfkristalle in einer Sorbitschmelze durch Rühren dispergiert werden und bei dem die Temperatur der Sorbitschmelze so eingestellt wird, dass die Schmelze nicht erstarrt und dass die Impfkristalle nicht schmelzen. Anschließend wird die mit Impfkristallen versetzte Schmelze während einer Zeit von mindestens 5 Minuten auf eine Temperatur von 50 bis 85 °C abgekühlt, wobei sich ein Feststoff bildet, der danach auf Raumtemperatur abgekühlt wird. Die aus Sorbit bestehenden Impfkristalle können Fette, Öle und/oder oberflächenaktive Substanzen enthalten und werden der Sorbitschmelze in einer Menge von 1 bis 50 Gew. -% zugesetzt.

Die DE-A 37 32 141 gibt einen Überblick über die zur Herstellung von festem Sorbit bekannten Verfahren. Bei einem dieser Verfahren wird die Sorbit-Lösung bis zur fast wasserfreien Schmelze im Vakuum eingedampft. Beim Abkühlen, eventuell unter Zugabe von kristallinem Sorbit, erstarrt die Schmelze, und durch Brechen und Mahlen wird aus der erstarrten Schmelze körniger, staubförmiger Sorbit erhalten. Das so hergestellte Produkt besitzt aber ein sehr breites Kornspektrum und der Anteil an γ-Sorbit unterliegt großen Schwankungen. Neben kristallinem Sorbit sind beträchtliche Mengen amorphen Sorbits enthalten. Dadurch ergibt sich ein schlechtes Rieselverhalten, und bei der Lagerung verklumpt das Produkt zu einer harten Masse. Nach einem weiteren bekannten Verfahren wird eine konzentrierte Sorbit-Lösung auf ein mechanisch bewegtes Bett von kristallisiertem Sorbit getropft oder grob versprüht. Das bewegte und benetzte Kristallbett wird zur Verdampfung des Wassers unter Durchleiten von Luft oder Inertgas auf einer Temperatur von 60 bis 80°C gehalten. Nach einer entsprechenden Kristallisationszeit wird unter Abkühlung kontinuierlich ein Teil des festen Sorbits entnommen. Das so erhaltene Produkt weist ebenfalls ein sehr breites Kornspektrum auf. Von Nachteil ist auch, daß die einzelnen Sorbit-Teilchen bei geringer Feuchtigkeitsaufnahme aus der Luft schnell zu harten Massen verbacken und daß die Lösezeit in Wasser relativ hoch liegt. Schließlich ist ein weiteres Verfahren bekannt, bei dem eine 50- bis 80%ige gereinigte Sorbit-Lösung kontinuierlich über eine Sprühvorrichtung in feinste Tröpfchen zerlegt und gleichzeitig feinteiliger, kristallisierter Sorbit mit Luft so in einen Sprühturm eingetragen wird, daß die versprühte Sorbit-Lösung die kristallinen Sorbit-Partikel mit einem dünnen Film überzieht. Zur Wasserverdampfung wird zusätzlich ein Warmluftstrom eingeblasen. Die Restfeuchte kann durch die Warmlufttemperatur und durch das Verhältnis von Warmluft zu Sorbit-Lösung in relativ weiten Grenzen variiert werden. Nach einer Kristallisationszeit von 20 bis 90 Minuten wird das trockene Produkt auf eine Temperatur < 40°C abgekühlt und ein Teil des Produkts wird entnommen, während der andere Teil in den Sprühturm zurückgeführt wird. Auch die Gebrauchseigenschaften des nach diesem Verfahren hergestellten kristallinen Sorbits sind letztlich nicht befriedigend. Um die Nachteile der nach den bekannten Verfahren hergestellten Produkte zu vermeiden, schlägt die DE-A 37 32 141 ein Verfahren zur Herstellung von kristallinem Sorbit mit verbesserten anwendungstechnischen Eigenschaften vor, das nach dem Prinzip der Sprühtrocknung arbeitet, wobei dem Sprühtrockner eine 50- bis 80%ige wäßrige Sorbit-Lösung und kristalliner Sorbit zugeführt werden.

Der Erfindung liegt die Aufgabe zugrunde, ein Verfahren zur Herstellung von kristallinem D-Sorbit zu schaffen, das nach dem Prinzip der Schmelzkristallisation arbeitet, das aber die bekannten Nachteile der Schmelzkristallisation vermeidet. Der nach der Erfindung hergestellte kristalline D-Sorbit soll einen Schmelzpunkt von 98 bis 100°C, einen Wassergehalt < 0,5 Gew% und einen Gehalt an γ-Modifikation > 90 % aufweisen. Ein derartiges Produkt hat optimale Gebrauchseigenschaften bei seiner Verwendung als industrieller Rohstoff. Daher ist das Herstellungsverfahren, das nach dem Prinzip der Schmelzkristallisation mit vorgeschalteter Vakuumeindampfung arbeitet, so weiter zu bilden bzw. zu verbessern, daß die Anforderungen an die Produktqualität sicher und über einen längeren Betriebszeitraum zuverlässig eingehalten werden, wobei außerdem eine wirtschaftliche Arbeitsweise erforderlich ist.

Die der Erfindung zugrundeliegende Aufgabe wird dadurch gelöst, daß eine D-Sorbit-Schmelze mit einem Wassergehalt < 0,5 Gew% und einer Temperatur, die 5 bis 15°C oberhalb des Erstarrungspunktes der Schmelze liegt, in einem Schmelzkristallisator kristallisiert wird, der aus einem gekühlten Behälter und einer waagerecht angeordneten, drehbaren, mit Scheibenelementen und Mischbarren bestückten Welle besteht, dessen Scheibenelemente und/oder Mischbarren in Form einer Schnecke auf der Welle angeordnet sind und bei dem die Scheibenelemente, die Mischbarren und/oder die Welle gekühlt werden und daß die dem Schmelzkristallisator entnommenen Kristalle durch Mahlung und Siebung zu einem Endprodukt verarbeitet werden, das zu > 90 % aus kristallinem D-Sorbit der γ- Modifikation besteht.

Durch die erfindungsgemäße Schmelzkristallisation entfällt die Notwendigkeit einer Zugabe von festem D-Sorbit zur D-Sorbit-Schmelze, denn durch die erfindungsgemäße Gestaltung der Schmelzkristallisation werden Kristallisationskeime in ausreichender Menge gebildet. Durch den relativ hohen mechanischen Energieeintrag, der durch die erfindungsgemäß zu verwendenden Mischorgane erfolgt, werden Kristalle mit geringer mittlerer Teilchengröße erzeugt, in denen nur eine sehr geringe Menge der Schmelze eingeschlossen ist, so daß das Endprodukt nicht merklich durch amorphen D-Sorbit verunreinigt ist. Beim erfindungsgemäßen Verfahren kann daher in vorteilhafter Weise auf eine Nachreifung des Produkts, also die Umwandlung von amorphem oder in der α- und β-Modifikation vorliegendem D-Sorbit in die γ-Modifikation, verzichtet werden. Die erfindungsgemäße Schmelzkristallisation bewirkt ferner in vorteilhafter Weise, daß der Aufwand für die Mahlung niedrig gehalten werden kann. Schließlich wird durch die nach der Erfindung vorgesehene Kühlung der einzelnen Teile des Schmelzkristallisators ein sehr günstiger Verlauf der Schmelzkristallisation erreicht, die dazu führt, daß das Endprodukt einen Gehalt an γ-Modifikation > 90 % aufweist, wodurch die Nachreifung des Produkts entfallen kann.

Nach der Erfindung hat es sich als besonders vorteilhaft erwiesen, wenn eine wässrige Lösung mit einem D-Sorbit-Gehalt von 60 bis 80 Gew% durch Eindampfung bei 5 bis 200 mbar, vorzugsweise 50 bis 200 mbar, und 130 bis 170°C, vorzugsweise 130 bis 140°C, in eine D-Sorbit-Schmelze mit einem Wassergehalt < 0,5 Gew% überführt wird und wenn die D-Sorbit-Schmelze auf eine Temperatur abgekühlt wird, die 5 bis 15°C, vorzugsweise 10 bis 15°C, oberhalb des Erstarrungspunktes der Schmelze liegt. Durch die erfindungsgemäße Eindampfung wird eine sehr weitgehende Entwässerung erreicht, so daß die zur Abkühlung der D-Sorbit-Schmelze zu leistende Kühlarbeit in vorteilhafter Weise relativ gering ist.

Nach der Erfindung hat es sich ferner als besonders vorteilhaft erwiesen, wenn die Eindampfung der wäßrigen D-Sorbit-Lösung in einem Dünnschichtverdampfer erfolgt, denn in diesem Apparat können die Eindampfbedingungen sehr genau eingehalten werden, und die Entwässerung wird in relativ kurzer Zeit erreicht.

In weiterer Ausgestaltung der Erfindung ist vorgesehen, daß die D-Sorbit-Schmelze auf eine Temperatur von 105 bis 115°C abgekühlt wird, daß die D-Sorbit-Schmelze im Schmelzkristallisator eine mittlere Verweilzeit von 1 bis 5 Stunden hat und daß die dem Schmelzkristallisator entnommenen Kristalle einen mittleren Teilchendurchmesser d₅₀ von < 2 mm aufweisen. Bei diesen Verfahrensbedingungen kann ein Endprodukt von hoher Qualität sicher erzeugt werden.

Schließlich ist nach der Erfindung vorgesehen, daß das Verfahren kontinuierlich durchgeführt wird, was sich insbesondere vorteilhaft auf seine Wirtschaftlichkeit auswirkt. Die notwendige Betriebssicherheit des kontinuierlich durchgeführten erfindungsgemäßen Verfahrens wird zuverlässig eingehalten.

Der Gegenstand der Erfindung wird nachfolgend anhand eines Verfahrensfließbildes näher erläutert.

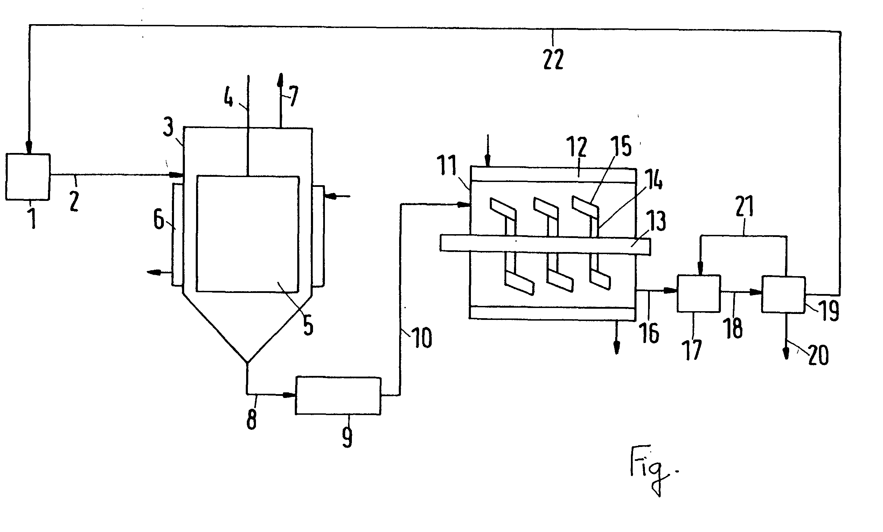

In einem Vorratstank (1) befindet sich eine wäßrige D-Sorbit-Lösung mit einem D-Sorbit-Gehalt von ca. 70 Gew.-%. Derartige Lösungen sind handelsüblich. Der D-Sorbit wird durch katalytische Hydrierung von D-Glukose in wäßriger Lösung hergestellt. Nach der Herstellung des D-Sorbits wird die wäßrige Lösung gereinigt und bis zu einem D-Sorbit-Gehalt von ca. 70 Gew.-% aufkonzentriert. Der Vorratstank (1) ist mit einem in der Zeichnung nicht dargestellten Rührer versehen.

Die wäßrige D-Sorbit-Lösung gelangt über die Leitung (2) kontinuierlich in den Dünnschichtverdampfer (3), der mit einem Rührer (4) ausgerüstet ist, welcher ein oder mehrere Wischerblätter (5) trägt. Die wäßrige D-Sorbit-Lösung rieselt in einem dünnen Film an der Wand des Dünnschichtverdampfers (3) herunter und wird dabei auf eine Temperatur von ca. 135°C durch das im Heizmantel (6) geführte Heizmedium aufgeheizt. Im Dünnschichtverdampfer (3) wird der wäßrigen D-Sorbit-Lösung das Wasser bis auf einen Restgehalt < 0,5 Gew% entzogen. Die Verdampfung des Wassers findet bei ca. 100 mbar statt, und der Wasserdampf wird aus dem Dünnschichtverdampfer (3) über die Leitung (7) abgeführt. Die im Dünnschichtverdampfer (3) erzeugte D-Sorbit-Schmelze wird von der Wand des Dünnschichtverdampfers (3) durch die Wischerblätter (5) kontinuierlich entfernt und fließt aus dem unteren Teil des Dünnschichtverdampfers (3) kontinuierlich über die Leitung (8) mit einer Temperatur von ca. 135°C in den Kühler (9), wo sie auf eine Temperatur von ca. 110 bis 115°C abgekühlt wird. Die D-Sorbit-Schmelze hat bei diesen Bedingungen gute Fließeigenschaften, so daß der kontinuierliche Betrieb nicht behindert ist. Die in der Leitung (8) fließende D-Sorbit-Schmelze wird auf Normaldruck gebracht, so daß der Kühler (9) bei Normaldruck arbeitet.

Über die Leitung (10) wird die abgekühlte D-Sorbit-Schmelze mit einer Temperatur von 110 bis 115°C kontinuierlich in den Schmelzkristallisator (11) eingebracht. Der Schmelzkristallisator (11) ist als zylindrischer Behälter ausgeführt, und er besitzt einen Kühlmantel (12), über den durch ein Kühlmedium Wärme abgeführt wird. Außerdem ist der Schmelzkristallisator (11) mit einer drehbaren Welle (13) ausgerüstet, auf der Scheibenelemente (14) und Mischbarren (15) angeordnet sind. Sowohl die Welle (13) als auch die Scheibenelemente (14) und die Mischbarren (15) werden von einem Kühlmedium durchströmt, so daß auch über diese Apparateteile Wärme aus dem Schmelzkristallisator (11) abgeführt wird. Die Mischbarren (15) sind auf den Scheibenelementen (14) schneckenförmig angeordnet, so daß die durch die Scheibenelemente (14) verursachte radiale Mischwirkung durch eine von den Mischbarren (15) verursachte achsiale Transportwirkung überlagert wird. Außerdem erfolgt durch die Scheibenelemente (14) und die Mischbarren (15) eine Zerkleinerung der im Schmelzkristallisator (11) gebildeten Kristallagglomerate. In der vorderen Zone des Schmelzkristallisators (11) geht die D-Sorbit-Schmelze in eine plastilinartige Masse über. In der mittleren Zone des Schmelzkristallisators (11) wird die weiter abgekühlte und bereits fester gewordene D-Sorbit-Masse durch den über die Mischorgane bewirkten mechanischen Energieeintrag aufgebrochen. In der hinteren Zone des Schmelzkristallisators (11) wird die aufgebrochene Masse weiter zerkleinert, und es kommt außerdem zu einer internen Zirkulation eines Teils des gebildeten kristallinen D-Sorbits in die mittlere und vordere Zone des Schmelzkristallisators (11). Der pulverförmige kristalline D-Sorbit wird über einen seitlich angeordneten Stutzen aus dem Schmelzkristallisator (11) abgeführt, in dem ein in der Höhe verstellbares Wehr angeordnet ist, über das die im Schmelzkristallisator (11) befindliche D-Sorbit-Menge geregelt werden kann. Der kristalline D-Sorbit verläßt den Schmelzkristallisator (11) mit einer Temperatur von 40 bis 60°C. Insbesondere die Mischbarren (15) sind dafür verantwortlich, daß es im Schmelzkristallisator (11) nicht zu Anbackungen kommt.

Der kristalline D-Sorbit gelangt über die Leitung (16) in die Mühle (17), wo eine Aufmahlung des Produkts stattfindet. Allerdings ist die Mahlarbeit relativ gering, da der kristalline D-Sorbit den Schmelzkristallisator (11) immer mit einem mittleren Teilchendurchmesser d₅₀ von < 2mm, in der Regel aber mit einem mittleren Teilchendurchmesser d₅₀ von < 1 mm verläßt. Das aufgemahlene Produkt wird über die Leitung (18) auf ein Sieb (19) geführt, dem das Endprodukt mit dem jeweils erforderlichen Kornspektrum über die Leitung (20) entnommen wird. Die Überkorn-Fraktion wird über die Leitung (21) in die Mühle (17) zurückgeführt, während die Unterkorn-Fraktion über die Leitung (22) in den Vorratstank (1) gelangt, wo sie in der wäßrigen D-Sorbit-Lösung unter Rühren aufgelöst wird.

Das über die Leitung (20) abgeführte Endprodukt hat einen Schmelzpunkt von ca. 98 bis 100°C, einen Wassergehalt von < 0,5 Gew% und einen Anteil an γ-Modifikation > 90 %, wobei in der Regel der Anteil der γ-Modifikation bei ca. 95 % liegt. Der nach dem erfindungsgemäßen Verfahren hergestellte kristalline D-Sorbit hat gute Gebrauchseigenschaften, denn er hat eine gute Rieselfähigkeit, und bei seiner Lagerung verkleben die einzelnen Kristalle untereinander nicht.

## Patentansprüche

1. Verfahren zur Herstellung von kristallinem D-Sorbit durch Eindampfung einer wäßrigen D-Sorbit-Lösung im Vakuum und nachfolgende Schmelzkristallisation der bei der Eindampfung erhaltenen D-Sorbit-Schmelze, dadurch gekennzeichnet, daß eine D-Sorbit-Schmelze mit einem Wassergehalt < 0,5 Gew% und einer Temperatur, die 5 bis 15°C oberhalb des Erstarrungspunktes der Schmelze liegt, in einem Schmelzkristallisator kristallisiert wird, der aus einem gekühlten Behälter und einer waagerecht angeordneten, drehbaren, mit Scheibenelementen und Mischbarren bestückten Welle besteht, dessen Scheibenelemente und/oder Mischbarren in Form einer Schnecke auf der Welle angeordnet sind und bei dem die Scheibenelemente, die Mischbarren und/oder die Welle gekühlt werden und daß die dem Schmelzkristallisator entnommenen Kristalle durch Mahlung und Siebung zu einem Endprodukt verarbeitet werden, das zu > 90 % aus kristallinem D-Sorbit der γ-Modifikation besteht.

2. Verfahren nach Anspruch 1, dadurch gekennzeichnet, daß eine wässrige Lösung mit einem D-Sorbit-Gehalt von 60 bis 80 Gew% durch Eindampfung bei 5 bis 200 mbar und 130 bis 170°C in eine D-Sorbit-Schmelze mit einem Wassergehalt < 0,5 Gew% überführt wird und daß die D-Sorbit-Schmelze auf eine Temperatur abgekühlt wird, die 5 bis 15°C oberhalb des Erstarrungspunktes der Schmelze liegt.

3. Verfahren nach Anspruch 2, dadurch gekennzeichnet, daß eine wässrige Lösung mit einem D-Sorbit-Gehalt von 60 bis 80 Gew% durch Eindampfung bei 50 bis 200 mbar und 130 bis 140°C in eine D-Sorbit-Schmelze mit einem Wassergehalt < 0,5 Gew% überführt wird und daß die D-Sorbit-Schmelze auf eine Temperatur abgekühlt wird, die 10 bis 15°C oberhalb des Erstarrungspunktes der Schmelze liegt.

4. Verfahren nach Anspruch 2 oder 3, dadurch gekennzeichnet, daß die Eindampfung der wäßrigen D-Sorbit-Lösung in einem Dünnschichtverdampfer erfolgt.

5. Verfahren nach den Ansprüchen 2 bis 4, dadurch gekennzeichnet, daß die D-Sorbit-Schmelze auf eine Temperatur von 105 bis 115°C abgekühlt wird.

6. Verfahren nach den Ansprüchen 1 bis 5, dadurch gekennzeichnet, daß die D-Sorbit-Schmelze im Schmelzkristallisator eine mittlere Verweilzeit von 1 bis 5 Stunden hat.

7. Verfahren nach den Ansprüchen 1 bis 6, dadurch gekennzeichnet, daß die dem Schmelzkristallisator entnommenen Kristalle einen mittleren Teilchendurchmesser d₅₀ von < 2 mm aufweisen.

8. Verfahren nach den Ansprüchen 1 bis 7, dadurch gekennzeichnet, daß es kontinuierlich durchgeführt wird.

## Claims

1. A process for the preparation of crystalline D-sorbitol by evaporation of an aqueous D-sorbitol solution in a vacuum and subsequent melt crystallisation of the D-sorbitol melt obtained during the evaporation, characterised in that a D-sorbitol melt having a water content of < 0.5% by weight and a temperature which is 5 to 15°C above the solidification point of the melt is crystallised in a melt crystalliser which consists of a cooled container and a horizontally arranged, rotatable shaft equipped with disc elements and mixing bars, the disc elements and/or mixing bars of which are arranged on the shaft in the form of a screw and in which the disc elements, the mixing bars and/or the shaft are cooled, and that the crystals withdrawn from the melt crystalliser are processed by grinding and sieving to obtain an end product which for more than > 90% consists of crystalline D-sorbitol of the y modification.

2. A process according to Claim 1, characterised in that an aqueous solution having a D-sorbitol content of 60 to 80% by weight is converted into a D-sorbitol melt having a water content of < 0.5% by weight by evaporation at 5 to 200 mbar and 130 to 170°C and that the D-sorbitol melt is cooled to a temperature which is 5 to 15°C above the solidification point of the melt.

3. A process according to Claim 2, characterised in that an aqueous solution having a D-sorbitol content of 60 to 80% by weight is converted into a D-sorbitol melt having a water content of < 0.5% by weight by evaporation at 5 to 200 mbar and 130 to 140°C and that the D-sorbitol melt is cooled to a temperature which is 10 to 15°C above the solidification point of the melt.

4. A process according to Claim 2 or 3, characterised in that the evaporation of the aqueous D-sorbitol solution is effected in a film evaporator.

5. A process according to Claims 2 to 4, characterised in that the D-sorbitol melt is cooled to a temperature of 105 to 115°C.

6. A process according to Claims 1 to 5, characterised in that the D-sorbitol melt has an average dwell time of 1 to 5 hours in the melt crystalliser.

7. A process according to Claims 1 to 6, characterised in that the crystals removed from the melt crystalliser have an average particle diameter d₅₀ of < 2 mm.

8. A process according to Claims 1 to 7, characterised in that it is performed continuously.

## Revendications

1. Procédé de préparation de D-sorbitol cristallin par évaporation d'une solution aqueuse de D-sorbitol sous vide et ensuite cristallisation à l'état fondu du D-sorbitol fondu obtenu lors de l'évaporation, caractérisé en ce qu'il consiste à cristalliser une masse fondue de D-sorbitol ayant une teneur en eau < 0,5% en poids et une température qui est supérieure de 5 à 15°C au point de solidification de la masse fondue dans un cristallisoir pour masse fondue constitué d'un récipient refroidi et d'un arbre horizontal pouvant tourner, ayant des éléments de disque et équipé de barres de mélange dont les éléments de disque et/ou les barres de mélange sont disposés sous la forme d'une vis sur l'arbre et dans lequel les éléments de disque, les barres de mélange et/ou l'arbre sont refroidis, et à transformer les cristaux prélevés du cristallisoir de masse fondue par broyage et tamisage en un produit fini qui est constitué pour plus de 90% de D-sorbitol cristallin de forme γ.

2. Procédé suivant la revendication 1, caractérisé en ce qu'il consiste à transformer une solution aqueuse ayant une teneur en sorbitol D de 60 à 80% en poids par évaporation entre 5 et 200 mbars et entre 130 et 170°C en une masse fondue de D-sorbitol ayant une teneur en eau < 0,5% en poids et à refroidir la masse fondue de D-sorbitol à une température qui est supérieure de 5 à 15°C au point de solidification de la masse fondue.

3. Procédé suivant la revendication 2, caractérisé en ce qu'il consiste à transformer une solution aqueuse ayant une teneur en sorbitol D de 60 à 80% en poids par évaporation entre 50 et 200 mbars et entre 130 et 140°C en une masse fondue de D-sorbitol ayant une teneur en eau < 0,5% en poids et à refroidir la masse fondue de D-sorbitol à une température qui est supérieure de 10 à 15°C au point de solidification de la masse fondue.

4. Procédé suivant la revendication 2 ou 3, caractérisé en ce qu'il consiste à effectuer l'évaporation de la solution aqueuse de D-sorbitol dans un évaporateur à couche mince.

5. Procédé suivant les revendications 2 à 4, caractérisé en ce qu'il consiste à refroidir la masse fondue de D-sorbitol à une température de 105 à 115°C.

6. Procédé suivant les revendications 1 à 5, caractérisé en ce que la masse fondue de D-sorbitol a dans le cristallisoir de masse fondue une durée moyenne de séjour de 1 à 5 heures.

7. Procédé suivant les revendications 1 à 6, caractérisé en ce que les cristaux prélevés du cristallisoir de masse fondue ont un diamètre d₅₀ moyen de particules < 2 mm.

8. Procédé suivant les revendications 1 à 7, caractérisé en ce qu'il est effectué en continu.
